# EUROPÄISCHE PATENTANMELDUNG

(11) **EP 4 796 071 A1**
(43) Veröffentlichungstag der Anmeldung: **26.08.2026**
(21) Anmeldenummer: 26151474.9
(22) Anmeldetag: 13.01.2026
(51) Int. Cl.: A61B 90/70, A61C 19/00, A61L 2/18, A61L 2/22, A61L 2/24, A61B 90/00

(54) **REINIGUNGSGERÄT UND VERFAHREN ZUM BETREIBEN EINES REINIGUNGSGERÄTS**

(30) Priorität: 20.02.2025 BE 202500019
(71) Anmelder: Miele & Cie. KG, 33332 Gütersloh (DE)
(72) Erfinder: Deppe, Fabian Lennart, 32107 Bad Salzuflen (DE); Wetzlar, Linus, 49324 Melle (DE)

(57) **Zusammenfassung**

Ein Reinigungsgerät (100) für medizinische Instrumente umfasst einen Reinigungsraum (110) zum Aufnehmen der medizinischen Instrumente, eine Aufnahme (170) zum Aufnehmen einer Druckdose (145), die ein Pflegemittel zum Pflegen der medizinischen Instrumente bevorratet, wobei die Aufnahme (170) ausgeformt ist, um ein Dosenventil der Druckdose (145) offenzuhalten, wenn die Druckdose (145) von der Aufnahme (170) aufgenommen ist, eine Leitung (150), die mit der Aufnahme (170) verbunden ist, um das Pflegemittel zu dem Reinigungsraum (110) zu leiten, ein Ventil (220), das in der Leitung (150) angeordnet ist, wobei ein Öffnungszustand des Ventils (220) durch ein Steuersignal steuerbar ist, einen Drucksensor (230), der zwischen der Aufnahme (170) und dem Ventil (220) mit der Leitung (150) gekoppelt ist, und welcher dazu ausgeformt ist, um ein Drucksignal auszugeben, das einen in der Leitung (150) vorhandenen Druck repräsentiert. Eine Steuereinrichtung ist ausgebildet, um unter Verwendung des Drucksignals das Steuersignal bereitzustellen.

## Beschreibung

Die Erfindung betrifft ein Reinigungsgerät und ein Verfahren zum Betreiben des Reinigungsgeräts für medizinische Instrumente.

Die WO 2011101396 A1 beschreibt ein Gerät, welches zum Desinfizieren, Sterilisieren und/oder Pflegen von ärztlichen Instrumenten vorgesehen ist.

Der hier vorgestellte Ansatz stellt sich die Aufgabe, ein verbessertes Reinigungsgerät für medizinische Instrumente und ein verbessertes Verfahren zum Betreiben des Reinigungsgeräts zu schaffen.

Erfindungsgemäß wird diese Aufgabe durch ein Reinigungsgerät und ein Verfahren zum Betreiben des Reinigungsgeräts mit den Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen in einer genauen Dosiermöglichkeit beim Leiten von Pflegemittel in das Reinigungsgerät. Somit kann verhindert werden, dass zu viel oder zu wenig Pflegemittel in das Reinigungsgerät, insbesondere zu den darin aufgenommenen medizinischen Instrumenten, geleitet wird.

Ein Reinigungsgerät für medizinische Instrumente weist folgende Merkmale auf:
einen Reinigungsraum zum Aufnehmen der medizinischen Instrumente;
eine Aufnahme zum Aufnehmen einer Druckdose, die ein Pflegemittel zum Pflegen der medizinischen Instrumente bevorratet, wobei die Aufnahme ausgeformt ist, um ein Dosenventil der Druckdose offenzuhalten, wenn die Druckdose von der Aufnahme aufgenommen ist;
eine Leitung, die mit der Aufnahme verbunden ist, um das Pflegemittel zu dem Reinigungsraum zu leiten;
ein Ventil, das in der Leitung angeordnet ist, wobei ein Öffnungszustand des Ventils durch ein Steuersignal steuerbar ist;
einen Drucksensor, der zwischen der Aufnahme und dem Ventil mit der Leitung gekoppelt ist, und welcher dazu ausgeformt ist, um ein Drucksignal auszugeben, das einen in der Leitung vorhandenen Druck repräsentiert; und
eine Steuereinrichtung, die ausgebildet ist, um unter Verwendung des Drucksignals das Steuersignal bereitzustellen. Gemäß einer Ausführungsform kann das Reinigungsgerät als ein Reinigungs- und Desinfektionsgerät ausgeformt sein. Insbesondere kann das Reinigungsgerät als ein Dentalpflegegerät ausgeformt sein. Die medizinischen Instrumente können in dieser Ausführungsform beispielsweise Geräte zur Dentalbehandlung sein. Das Reinigungsgerät kann somit zur Reinigung verschiedener medizinischer Instrumente verwendet werden, die auch als Übertragungsinstrumente bezeichnet werden, und beispielsweise Turbinen oder Hand- und Winkelstücke umfassen. Der Reinigungsraum kann dabei ein Raum sein, in welchem verschiedene Schritte oder Anwendungen zum Reinigen und Pflegen der medizinischen Instrumente stattfinden. Die Druckdose kann dabei als eine Pflegemitteldose ausgeformt sein, welche Pflegemittel für das Reinigungsgerät beinhaltet. Die Druckdose weist dabei wie übliche Druckdosen oben den Ventilbund und das Dosenventil auf. Der Ventilbund kann beispielsweise ein zylinderförmiger oder kegelförmiger Bereich der Druckdose zwischen dem Dosenventil und einem Hauptkörper der Druckdose angeordnet sein. Das Dosenventil kann dabei zu einer Ausgabe des Pflegemittels dienen. Das Ventil kann dabei in der Leitung angeordnet sein, und nur Pflegemittel durchlassen, wenn das Steuersignal anliegt. Somit kann unter Verwendung des Ventils trotz dauerhaft geöffnetem Dosenventil reguliert werden, wann das Pflegemittel ausgegeben wird. Der Drucksensor kann ausgebildet sein, um zu messen, welcher Druck in der Druckdose vorhanden ist. Dazu kann der Drucksensor der Druckdose nachgeschaltet sein. Die Steuereinrichtung kann ausgebildet sein, um das Steuersignal abhängig von einem Wert des von dem Drucksensor gemessenen Drucks zu generieren. Dabei kann eine Charakteristik des Steuersignals abhängig von einer Größe des Werts des gemessenen Drucks eingestellt werden. Beispielsweise kann bei einem geringen gemessenen Druck das Steuersignal mit einer ersten Charakteristik bereitgestellt werden, die einen längeren Öffnungszustand und/oder größeren Öffnungsquerschnitt des Ventils bewirkt. Bei einem großen gemessenen Druck kann das Steuersignal im Vergleich dazu mit einer zweiten Charakteristik bereitgestellt werden, die einen im Vergleich dazu kürzeren Öffnungszustand und/oder kleineren Öffnungsquerschnitt des Ventils bewirkt. Auf diese Weise kann das Steuersignal abhängig von der Größe des gemessenen Drucks so generiert werden, dass unabhängig von der Größe des gemessenen Drucks zumindest annähernd die gleiche Menge an Pflegemittel durch das Ventil geleitet wird. Die hier genannten Ventile des Reinigungsgeräts können als Magnetventile ausgeführt sein.

Die Steuereinrichtung kann dabei ausgebildet sein, um das Steuersignal zum Öffnen des Ventils für eine von der Größe des Drucksignals abhängigen Zeitdauer bereitzustellen. Somit kann die Zeitdauer der Öffnung des Ventils abhängig von der Größe des gemessenen Drucks präziser festgelegt werden.

Beispielsweise kann die Steuereinrichtung ausgebildet sein, um das Steuersignal zum Öffnen des Ventils für eine erste Zeitdauer bereitzustellen, wenn das Drucksignal einen ersten Druck repräsentiert. Entsprechend kann die Steuereinrichtung ausgebildet sein, um das Steuersignal zum Öffnen des Ventils für eine zweite Zeitdauer bereitzustellen, wenn das Drucksignal einen zweiten Druck repräsentiert. Dabei kann der erste Druck größer als der zweite Druck und die erste Zeitdauer kleiner als die zweite Zeitdauer sein. Somit kann ein Öffnungsintervall des Ventils größer sein, wenn der Drucksensor einen niedrigen Druck misst und das Öffnungsintervall des Ventils kann größer sein, wenn der Drucksensor einen hohen Druck misst. Das kann bedeuten, dass bei jedem Pflegeprozess gleich viel Pflegemittel verwendet wird, indem ein Druckniveau in der Druckdose berücksichtigt wird. Die erste Zeitdauer kann dabei beispielsweise zwei Sekunden und die zweite Zeitdauer sechs Sekunden betragen.

Das Reinigungsgerät kann ein Rückschlagventil haben, wobei das Rückschlagventil dazu ausgeformt ist, um zu verhindern, dass das Pflegemittel in die Aufnahme gelangt, etwa bei Abkopplung der Druckdose. Das Rückschlagventil kann dazu in der Leitung zwischen der Aufnahme und dem Drucksensor angeordnet sein.

Die Leitung kann beispielsweise innerhalb des Reinigungsraums mit einer Mehrzahl an Anschlüssen für die medizinischen Instrumente verbunden sein. Die Mehrzahl an Anschlüssen kann dabei dazu da sein, um das Pflegemittel in die medizinischen Instrumente einzuspritzen. Dadurch kann eine Lebensdauer von medizinischen Instrumenten verlängert werden.

Gemäß einer Ausführungsform kann die Leitung sich zumindest einmal innerhalb des Reinigungsraums teilen oder verzweigen, um das Pflegemittel an die Mehrzahl an Anschlüssen für die medizinischen Instrumente zu fördern. Dabei kann sich die Leitung beispielsweise nur einmal teilen, alternativ kann die Leitung sich auch mehrmals teilen, sodass beispielsweise mehr als vier Anschlüsse vorgesehen sein können.

Das Reinigungsgerät kann einen Druckluftanschluss haben, wobei die Leitung über ein Druckluftventil mit dem Druckluftanschluss verbunden ist. Der Druckluftanschluss kann beispielsweise das Pflegemittel durch die Leitung fördern, wenn das Druckluftventil geöffnet wird. Dabei kann das Druckluftventil ein Magnetventil sein.

Außerdem kann das Reinigungsgerät ein Umwälzsystem zum Umwälzen von Reinigungsmittel unter Verwendung einer Pumpe umfassen, die mittels einer weiteren Leitung mit der Mehrzahl an Anschlüsse für die medizinischen Instrumente verbunden ist. Somit können die medizinischen Instrumente vor einer Pflege unter Verwendung des Reinigungsmittels innerlich gesäubert werden.

Das Reinigungsgerät kann zudem ein Verbindungselement zum Verbinden des der Pumpe mit einer Korbankupplung für einen Sprüharm umfassen. Das Verbindungselement kann dabei als eine Leitung ausgeformt sein. Durch den Sprüharm kann das Spülmittel großflächig über den Reinigungsraum verteilt werden. Somit können die medizinischen Instrumente unter Verwendung des Reinigungsmittels äußerlich gesäubert werden

Ferner wird ein Verfahren zum Betreiben eines Reinigungsgeräts vorgestellt, wobei das Verfahren einen Schritt des Ausgebens des Drucksignals, das einen in der Leitung vorhandenen Druck repräsentiert, unter Verwendung des Drucksensors, der zwischen der Aufnahme und dem Ventil mit der Leitung gekoppelt ist, einen Schritt des Bereitstellens eines Steuersignals ansprechend auf das Drucksignal, unter Verwendung der Steuereinrichtung, und einen Schritt des Ansteuerns eines Öffnungszustand des Ventils, ansprechend auf das Steuersignal hat.

Der beschriebene Ansatz kann hier in einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Ausführungsbeispiels eines Reinigungsgeräts;
- Figur 2: eine weitere schematische Darstellung eines Ausführungsbeispiels eines Reinigungsgeräts;
- Figur 3: eine Detaildarstellung eines Ausführungsbeispiels einer Pflegeeinheit;
- Figur 4: ein Diagramm eines Ausführungsbeispiels eines Konzepts für einen Algorithmus;
- Figur 5: ein weiteres Diagramm eines Ausführungsbeispiels eines Konzepts für einen Algorithmus; und
- Figur 6: ein Ablaufdiagramm eines Ausführungsbeispiels eines Verfahrens zum Betreiben eines Reinigungsgeräts

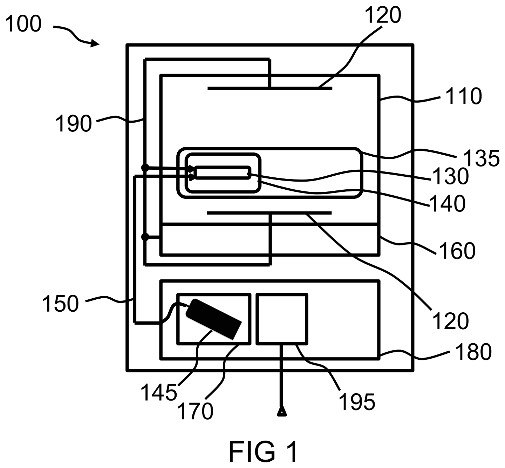

Figur 1 zeigt eine schematische Darstellung eines Ausführungsbeispiels eines Reinigungsgeräts 100. Das Reinigungsgerät 100 weist einen auch als Spülraum bezeichneten Reinigungsraum 110 auf, in dem medizinische Instrumente zur Reinigung angeordnet werden können. Beispielsweise ist in dem Reinigungsraum 110 zumindest ein Sprüharm 120 angeordnet, mit dem die medizinischen Instrumente mit einem

Reinigungsmittel besprüht werden können. Dadurch können die medizinischen Instrumente äußerlich gereinigt werden.

Ferner ist in dem Reinigungsraum 110 ein Medienverteiler 130 und ein Korb 135 mit einem Korbeinsatz 140 angeordnet. Die medizinischen Instrumente können von dem Korbeinsatz 140 gehalten werden und an den Medienverteiler 130 angeschlossen sein. Über den Medienverteiler 130 kann das Reinigungsmittel in ein Inneres der medizinischen Instrumente geleitet werden, um die medizinischen Instrumente von innen zu reinigen.

Das Reinigungsmittel wird gemäß einem Ausführungsbeispiel am Boden des Reinigungsraums 110 aufgefangen und während eines Reinigungsprozesses unter Verwendung einer Leitung 190 und eines Umwälzsystems 160 und Reinigungsleitungen erneut zu dem zumindest einen Sprüharm 120 und dem Medienverteiler 130 geführt.

Zum Pflegen der medizinischen Instrumente, beispielsweise zum Ende eines Reinigungsprozesses wird ein Pflegemittel verwendet, das in einer Druckdose 145 bevorratet wird. Gemäß einem Ausführungsbeispiel wird das Pflegemittel aus der Druckdose 145 über eine Leitung 150 zu dem Medienverteiler 130 geleitet. Über den Medienverteiler 130 wird das Pflegemittel gemäß einem Ausführungsbeispiel in ein Inneres der medizinischen Instrumente geleitet.

Beispielsweise umfasst das Reinigungsgerät 100 eine Aufnahme 170 für die Druckdose 145, welche beispielsweise in einer Pflege- und Drucklufteinheit 180 angeordnet ist. Die Aufnahme 170 wird auch als Pflegemittelaufnahme bezeichnet.

Gemäß einem Ausführungsbeispiel ist das Reinigungsgerät 100 ein Reinigungs- und Desinfektionsgerät und wird im Folgenden auch als solches bezeichnet. Gereinigt werden Utensilien erst mit einem Reinigungsmittel, und anschließend werden diese beispielsweise mit Wasserdampf desinfiziert.

Das Reinigungsgerät 100 ist außerdem dazu da, um verschiedene medizinische Instrumente oder auch andere Utensilien nach einem Reinigen und Desinfizieren zu Pflegen. Dabei wird das Pflegemittel, beispielsweise ein Pflegeöl, aus der Druckdose 145 entnommen.

Die Pflege- und Drucklufteinheit 180 ist beispielsweise als eine Schublade ausgeformt. Außerdem umfasst die Pflege- und Drucklufteinheit 180 optional eine Druckluftregelung 195 des Reinigungsgerät 100.

Gemäß dem hier gezeigten Ausführungsbeispiel weist das Reinigungsgerät 100 auch den Korbeinsatz 140 auf, in den Utensilien gelegt werden können. Der Medienverteiler 130 verteilt dabei das gewünschte Mittel an verschiedene von dem Korbeinsatz aufgenommene medizinische Instrumente, wie einem Dentalbohrer. Es wird Reinigungsmittel und anschließend ein Pflegemittel, beispielsweise ein Reinigungsöl über den Medienverteiler 130 in die in dem Korbeinsatz 140 angesteckten medizinischen Instrumente geleitet. Der Korbeinsatz 140 ist gemäß dem hier beschriebenen Ausführungsbeispiel als eine Art Pflegemodul ausgeformt, welches beispielsweise eine gezieltere Reinigung ermöglicht.

Vorteilhafterweise ist in der Leitung 150 ein steuerbares Ventil angeordnet, das eine Dosierung des aus der Druckdose 145 entnommenen Pflegemittels abhängig von einem innerhalb der Druckdose 145 herrschenden Drucks ermöglicht.

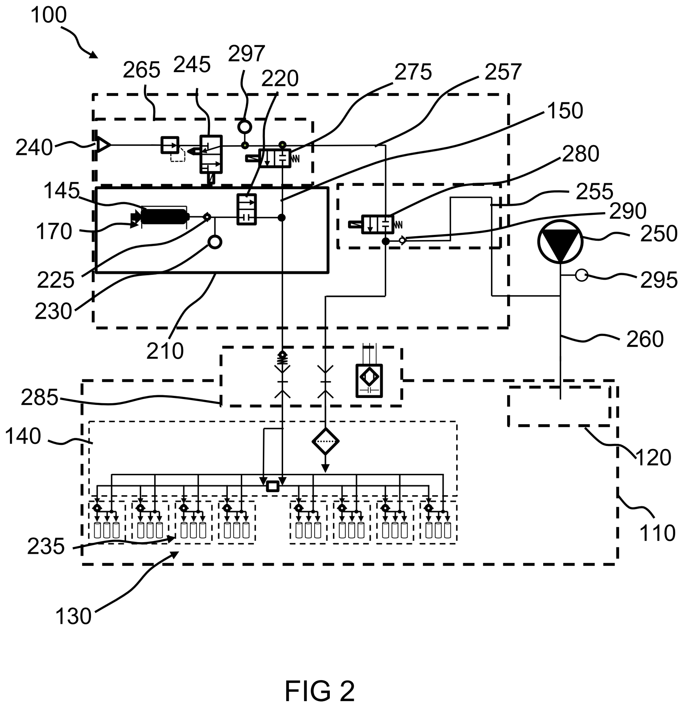

Figur 2 zeigt eine weitere schematische Darstellung eines Ausführungsbeispiels eines Reinigungsgeräts 100 für medizinische Instrumente. Das Reinigungsgerät 100 ist gemäß einem Ausführungsbeispiel wie das in Figur 1 beschriebene Reinigungsgerät ausgeformt.

Das Reinigungsgerät 100 umfasst eine Aufnahme 170 für die Druckdose 145 in einer Pflegeeinheit 210. An der Aufnahme 170 ist eine Leitung 150 mit einem Ventil 220, einem Rückschlagventil 225 und einem Drucksensor 230 angeordnet. Die Leitung 150 führt in den Reinigungsraum 110. Dabei endet die Leitung 150 an einem Medienverteiler 130, der eine Mehrzahl von Anschlüssen 235 umfasst. Über die Leitung 150 kann ein aus der Druckdose 145 entnommenes Pflegemittel über die Anschlüsse 235 zu den medizinischen Instrumenten geleitet werden. Vorteilhafterweise kann eine Entnahmemenge des Pflegemittels unter Verwendung des Ventils 220 unter Verwendung eines von dem Drucksensor 230 erfassten Drucks in der Leitung 150 so dosiert werden, dass bis zu einem vorbestimmten Entleerungszustand der Druckdose 145, unabhängig von dem in der Druckdose 145 herrschenden Drucks, eine vorbestimmte Menge an Pflegemittel zu den Anschlüssen geleitet werden kann.

Das Reinigungsgerät 100 umfasst ferner einen Druckluftanschluss 240, der über ein Druckluftventil 275 entweder mit der Leitung 150 oder über ein weiteres Ventil 280 mit dem Medienverteiler 130 gekoppelt werden kann. Auf diese Weise kann über den Druckluftanschluss 240 zugeführte Druckluft über die Anschlüsse 235 zu den medizinischen Instrumenten geleitet werden.

Außerdem weist das Reinigungsgerät 100 ein Umwälzsystem mit einer Pumpe 250 auf. Die Pumpe 250 ist über eine weitere Leitung 255 mit der Mehrzahl an Anschlüssen 235 verbunden und über ein Verbindungselement 260 mit einer Korbankupplung für zumindest einen Sprüharm 120 verbunden. Unter Verwendung der Pumpe 250 wird Reinigungsmittel zum innerlichen und äußerlichen Reinigen der medizinischen Instrumente gefördert.

Die in Figur 2 beschriebene Darstellung, beschreibt dabei eine Konkretisierung des Reinigungsgeräts 100. Gemäß einem Ausführungsbeispiel umfasst das Reinigungsgerät 100 die Pflegeeinheit 210, welche die Aufnahme 170 mit der Druckdose 145, einen Teil der Leitung 150, das Ventil 220, das Rückschlagventil 225 und den Drucksensor 230 umfasst. Dabei gibt die Druckdose 145 Pflegemittel über die Aufnahme 170 in die Leitung 150 aus. Das Pflegemittel ist dabei beispielsweise ein Weißöl. Durch das Rückschlagventil 225 wird verhindert, dass das Pflegemittel in die Aufnahme 170 gelangt, etwa bei Abkopplung der Druckdose. Der Drucksensor 230 wird verwendet, um einen Druck der Leitung 150 zu messen. Dabei ist der Drucksensor 230 zwischen dem Rückschlagventil 225 und dem Ventil 220 mit der Leitung 150 gekoppelt.

Einer Drucklufteinrichtung 265 umfasst den Druckluftanschluss 240, welcher Druckluft an das Reinigungsgerät 100 bereitstellt. Die Drucklufteinrichtung 265 umfasst außerdem das Druckluftventil 245, welches dazu verwendet wird, die Drucklufteinrichtung 265 entweder zu entlüften, oder um Druckluft an eine Druckluftleitung 257 weiterzuleiten. Das Druckluftventil 245 wird dabei beispielsweise ausgelöst über Stand-by 230V. Ein Druckluftventilventil 275, beispielsweise ein magnetisches Ventil, leitet anschließend die Druckluft entweder an die Leitung 150, oder an die Druckluftleitung 257. Wenn die Druckluft in die Leitung 150 geleitet wird, kann beispielsweise noch vorhandenes Pflegemittel in der Leitung 150 unter Verwendung der Druckluft über die Mehrzahl an Anschlüssen 235 ausgeblasen werden, um die Leitung 150 nach einem Pflegevorgang zu reinigen. Alternativ oder zusätzlich wird die Druckluft zu einem weiteren magnetischen Ventil 280 geleitet, wobei die Druckluft verwendet wird, um einen mit dem Medienverteiler 130 verbundenen Abschnitt der Druckluftleitung 257 nach einem Spülvorgang zu reinigen.

Sowohl die Leitung 150 sowie die weitere Leitung 255 durchlaufen dabei an einem Übergangsbereich zu dem Reinigungsraum 110 eine Ankopplung, beispielsweise in Form einer Dentalpflegeankopplung 285, welche den Reinigungsraum 110 mit einem außerhalb des Reinigungsraums 110 liegenden Abschnitt des Reinigungsgeräts 100 verbindet. Optional umfassen die Leitung 150 und/oder die weitere Leitung 255 mehrere weitere Rücklaufschlagventile 290 und Sensoriken 295, 297, beispielsweise in Form weiterer Drucksensoren.

Gemäß einem Ausführungsbeispiel ist eine Dentalpflegefunktion in dem auch als Reinigungs- und Desinfektionsgerät bezeichneten Reinigungsgerät 100 integriert. Dafür wird ein Pflegemittel benötigt, welches in die auch als Übertragungsinstrumente bezeichneten medizinischen Instrumente eingebracht wird. Das Pflegemittel dient der Schmierung der inneren Mechanik der medizinischen Instrumente. Dieses Pflegemittel wird in Form der unter Druck stehenden Druckdose 145 in dem Reinigungsgerät 100 adaptiert und das Dosenventil der Druckdose 145 wird dauerhaft betätigt. Mit dem nachgeschalteten steuerbaren Ventil 220 wird dann das Pflegemittel während eines Pflegeschritts freigegeben. Dabei kommt es bei immer gleichen Ölungsintervallen aufgrund der Verwendung des Drucksensors 230 zu denselben eingebrachten Ölmengen, die in den Instrumenten angelangen.

Durch die Aufnahme 170 und den Drucksensor 230 wird die eingebrachte Ölmenge durch eine Anpassung der Ölungsintervalle nahezu konstant gehalten. Dabei soll sichergestellt werden, dass die aufzubereitenden Übertragungsinstrumente aus wirtschaftlichen Gründen nicht zu viel Öl erhalten, trotzdem aber ausreichend geölt werden, um eine möglichst hohe Lebensdauer der Instrumente zu gewährleisten. Dies soll unabhängig von dem sich in der Öldose befindlichen Druck ermöglicht werden, welcher über die Nutzungsdauer hin abfällt.

Das Reinigungsgerät 100 umfasst eine druckgesteuerte Anpassung von Ölungsintervallen bei automatisierter Pflege von beispielsweise dentalen Übertragungsinstrumenten. Nach mehrmaliger Betätigung beziehungsweise Entnahme von Pflegemittel aus der Druckdose 145, kommt es zu einem Druckabfall innerhalb des Behälters der Druckdose 145. Das führt dazu, dass bei gleichbleibender Betätigungsdauer weniger Pflegemittel aus der Druckdose 145 gelangt. Dies kann durch eine geeignete Steuerung des Ventils 220 ausgeglichen werden, indem die Öffnungsdauer des Ventils 220 verlängert wird.

Bei der maschinellen Pflege der Übertragungsinstrumente wird gemäß dem hier beschriebenen Ansatz auf diesen Druckabfall reagiert. Andernfalls wäre zur Sicherstellung einer ausreichenden Ölung der Instrumente eine ständige Überölung notwendig, bis das niedrigste mögliche Druckniveau in der Dose erreicht ist, bei dem noch Öl gefördert wird, was zu einem unökonomischen Ölverbrauch führt und den Anwender dazu zwingt, die Öldosen öfter als eigentlich notwendig auszutauschen. Das hier beschriebene Reinigungsgerät 100 ist dazu ausgeformt, um neben der Reinigung, Desinfektion und zum Teil auch Sterilisation, die Übertragungsinstrumente zu ölen. Dazu werden Ölgebinde in Form von Kanistern, Tanks und, wie auch bei der manuellen Pflege, unter Druck stehende Öldosen, hier die Druckdose 145, genutzt.

Die Druckdose 145 wird an dem Reinigungsgerät 100 adaptiert und gibt bei betätigtem Ventil 220 das Pflegemittel, beispielsweise das Pflegeöl, für die Aufbereitung frei. Bei einem solchen Pflegeautomaten ist durch den Drucksensor 230 eine Zählung der Anzahl der Pflegezyklen nicht mehr notwendig. Auch eine frühzeitige Aufforderung, um die Druckdose 145 auszutauschen, um weiterhin eine sichere Schmierung zu gewährleisten, entfällt durch eine Verwendung des Drucksensors 230, und es muss die Druckdose 145 erst dann ausgetauscht werden, wenn diese auch tatsächlich leer ist.

Durch den Drucksensor 230 ist bei jedem Abzug die gleiche Menge an Pflegemittel erhalten, auch wenn die Druckdose 145 fast voll oder fast leer ist. Dadurch wird hier keine zwangsläufig unwirtschaftliche Überölung der Instrumente stattfinden. Andernfalls findet auch in den letzten Pflegevorgängen bei weniger Druck in der Druckdose 145 keine Unterölung statt. Beispielsweise ist die Druckdose 145 innerhalb einer Tür oder einer Schublade des Reinigungsgeräts 100, beispielsweise eines Sterilisators, eingesetzt. Sie wird beispielsweise mit einem bajonettartigen Verschluss gegen einen Ventilsitz gedrückt, wodurch das Dosenventil betätigt wird.

Durch das Reinigungsgerät 100 entsteht der Vorteil, dass bei der Bereitstellung des Pflegemittels für den Pflegeprozess nicht mehr davon ausgegangen werden muss, dass bei einem niedrigen Dosendruck noch genügend Pflegeöl zu den Instrumenten gefördert wird, um sie ordnungsgemäß zu schmieren. Es bedeutet, dass keine Überölung durch einen erhöhten Dosendruck eintritt. Diese bringt bei der Pflege der Instrumente einen erheblichen Mehrwert und ist zudem wirtschaftlich, da der Anwender die Druckdose 145 nicht öfter als notwendig wechseln muss.

Der Mehrwert besteht darin, dass es zu keiner Zeit zu einer zu geringen Schmierung der Adaptierten Übertragungsinstrumente kommt. Das bedeutet für den Anwender eine gewährleistete hohe Lebensdauer seiner Übertragungsinstrumente, was dem Anwender Neuanschaffungskosten einspart. Zudem ist die Überölung der Übertragungsinstrumente minimiert, was einen längeren Zeitraum bis zum Auswechseln der Druckdose 145 ermöglicht und dem Anwender somit einen wirtschaftlichen Vorteil verschafft.

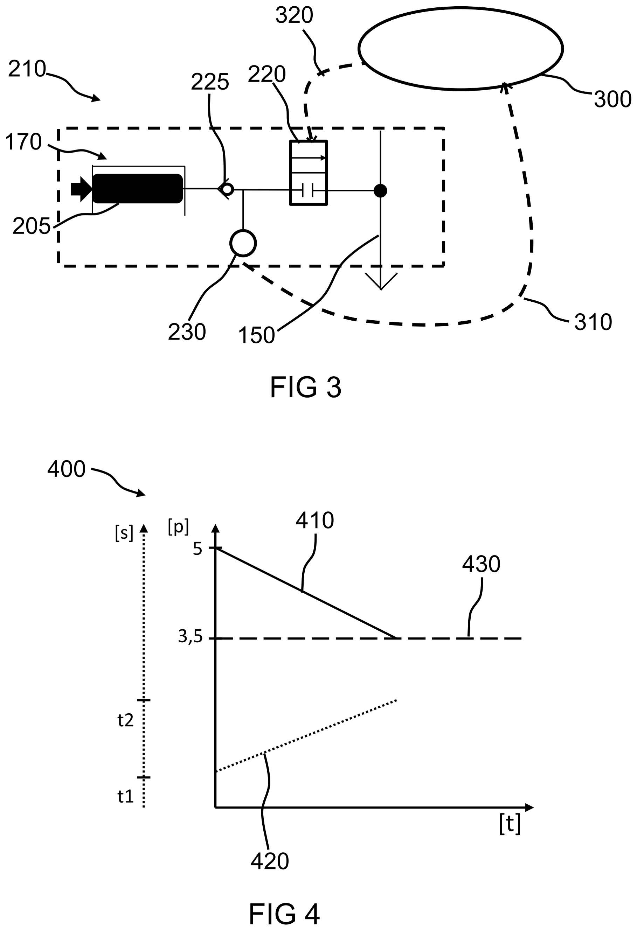

Figur 3 zeigt eine Detaildarstellung eines Ausführungsbeispiels einer Pflegeeinheit 210 und einer Steuereinrichtung 300, die beispielsweise Bestandteile des anhand von Fig. 2 beschriebenen Reinigungsgeräts sind. Beispielsweise ist die Steuereinrichtung 300 Bestandteil einer Gerätesteuerung.

Die Pflegeeinheit 210 umfasst auch hier die Aufnahme 170 mit der Druckdose 145, das Rückschlagventil 225, den Drucksensor 230 und das Ventil 220. Der Drucksensor 230 stellt dabei ein Drucksignal 310 bereit, welches an die Steuereinrichtung 300 geleitet wird. Die Steuereinrichtung 300 ist ausgebildet, um abhängig vom Druckwert, den das Drucksignal 310 repräsentiert, ein Steuersignal 320 zu generieren und an das Ventil 220 auszugeben, um einen Betrieb des Ventils 220 durch das Steuersignal 320 zu steuern.

Wenn das Drucksignal 310 beispielsweise einen ersten Druck, hier einen höheren Druck, repräsentiert, steuert die Steuereinheit 300 das Ventil 220 für eine kürzere Zeit als im Durchschnitt an, um zu versichern, dass nicht zuviel Pflegemittel in das Reinigungsgerät geleitet wird. Wenn das Drucksignal 310 einen zweiten Druck repräsentiert, welcher niedriger ist als der hohe erste Druck oder der Durchschnitt, dann steuert die Steuereinheit 300 das Ventil 220 länger an. Somit wird verhindert, dass zu wenig Pflegemittel ausgeleitet wird. Dabei ist die Druckdose 145 für eine Ölversorgung des Reinigungsgerätes ausgeformt, und das Pflegemittel wird in Richtung des Reinigungsraums gefördert. Dieser Ausschnitt einer Konzeptlandkarte zeigt, dass die Leitung 150 verwendet wird, um das Pflegemittel aus der Druckdose 145, die auch als Ölgebinde bezeichnet wird, in den Reinigungsraum, also in Richtung der Instrumente, zu leiten.

Die Druckdose 145 ist so in dem Reinigungsgerät 100 adaptiert, dass das Dosenventil ständig betätigt ist und an dem Ventil 220, welches das Pflegemittel für den Prozess freigibt, immer der Dosendruck anliegt. Die unter Druck stehende Druckdose 145 wird durch den Drucksensor 230 überwacht. Dieser ist der Dosenadaption nachgeschaltet und befindet sich somit zwischen der Öldosenadaption und dem Ventil 220, welches das Öl freigibt. Der Drucksensor 230 gibt den jeweiligen Dosendruck an die Gerätesteuerung aus. Diese steuert dann die Dauer, wie lange das Ventil 220, welches das Pflegemittel freigibt, während des Pflegeprozesses geöffnet bleibt. Je niedriger das Druckniveau in der Druckdose 145 ist, desto länger wird das Ventil 220 geöffnet, um immer auf ein konstantes eingebrachtes Pflegemittelvolumen zu kommen.

Figur 4 zeigt ein Diagramm 400 eines Ausführungsbeispiels eines Konzepts für einen Algorithmus für den hier beschriebenen Ansatz. Auf der Abszisse ist die Zeit in Sekunden und auf der Ordinate der Druck in bar aufgetragen. Auf einer zweiten gepunktet dargestellten Ordinate ist die Zeit in Sekunden aufgetragen.

In Figur 4 ist eine Gesamtölungszeit 410 dargestellt, die zwischen einem vollen Zustand der Druckdose, beispielsweise mit 5 bar und einer Entleerungsgrenze, beispielsweise bei 3,5 bar, verläuft.

Ferner ist eine Dauer eines Ölungsintervalls 420 dargestellt, das beispielsweise zwischen einer minimalen Dauer t1 und einer maximalen Dauer t2 variiert. Das Ölungsintervall 420 entspricht beispielsweise einer Öffnungsdauer des Ventils.

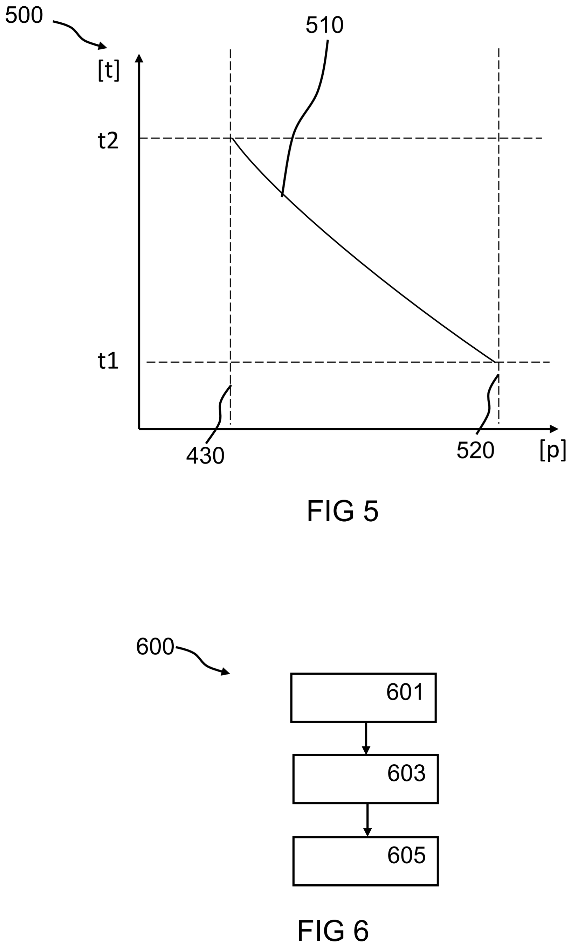

Figur 5 zeigt ein weiteres Diagramm 500 eines Ausführungsbeispiels eines Konzepts für einen Algorithmus für den hier beschriebenen Ansatz. Auf der Abszisse ist der Druck in bar und auf der Ordinate die Zeit in Sekunden aufgetragen.

In dieser gegenüber Figur 4 leicht verbesserten Darstellung wird ein Graph 510 gezeigt, welcher eine Dauer des Ölungsintervalls über den Druck darstellt. Aus dem Graph 510 ist ersichtlich, dass ein niedrigerer Dosendruck ein längeres Öffnungsintervall benötigt, um die gleiche Menge an Pflegemittel auszugeben, als wenn der Dosendruck höher ist. Die Entleerungsgrenze 430 liegt hierbei beispielsweise bei 3,5 bar, wobei ein Maximaldruck 520 beispielsweise bei 5 bar liegt. Bei der Entleerungsgrenze 430 beträgt die Dauer des Öffnungsintervalls eine Zeit t2 und bei dem Maximaldruck eine Zeit t1, die kleiner als t2 ist. Ausgehend von dem Maximaldruck erhöht sich die Dauer des Öffnungsintervalls kontinuierlich mit fallendem Druck in der Druckdose, bis die Entleerungsgrenze erreicht ist.

Figur 6 zeigt ein Ablaufdiagramm eines Ausführungsbeispiels eines Verfahrens 600 zum Betreiben eines Reinigungsgeräts, wie es anhand der vorangegangenen Figuren beispielhaft beschrieben ist.

Das Verfahren 600 umfasst einen Schritt 601 des Ausgebens des Drucksignals, das einen in der an die Druckdose angeschlossenen Leitung vorhandenen Druck repräsentiert. Ansprechend auf das Drucksignal wird ein Schritt 603 des Bereitstellens durchgeführt, in dem ein Steuersignal unter Verwendung des Drucksignals bereitgestellt wird. Das Steuersignal wird verwendet, um in einem Schritt 605 des Ansteuerns einen Öffnungszustand des Ventils zu steuern.

Das Verfahren 600 ermöglicht einen Ausgleich des Druckabfalls innerhalb der Druckdose während der Pflegeprozesse. innerhalb eines Reinigungs- und Desinfektionsgeräts, welches die automatisierte Aufbereitung und Pflege von medizinischen Instrumenten, beispielsweise dentalen Übertragungsinstrumenten, ermöglicht. Die Pflege beinhaltet hierbei beispielsweise die Ölung der innenliegenden Kanäle der medizinischen Instrumente mittels eines Pflegemittels, beispielsweise eines Pflegeöls. Eine entsprechende Druckdose ist sowohl bei der manuellen als auch der maschinellen und automatisierten Pflege von dentalen Übertragungsinstrumenten einsetzbar und ist beispielsweise als etablierte Sprühdose realisiert. Dabei handelt es sich um unter Druck stehende Behälter, welche mit einem Treibgas und dem Pflegemittel befüllt sind. Bei dem Pflegemittel handelt es sich beispielsweise um ein medizinisches Weißöl. Dieses wird bei Betätigung des Dosenventils freigegeben und gelangt durch die Düse der Druckdose bei der automatisierten Pflege in einen Pflegeautomaten und in das oder die medizinischen Instrumente.

## Patentansprüche

1. Reinigungsgerät (100) für medizinische Instrumente, wobei das Reinigungsgerät (100) folgende Merkmale aufweist:
einen Reinigungsraum (110) zum Aufnehmen der medizinischen Instrumente;
eine Aufnahme (170) zum Aufnehmen einer Druckdose (145), die ein Pflegemittel zum Pflegen der medizinischen Instrumente bevorratet, wobei die Aufnahme (170) ausgeformt ist, um ein Dosenventil der Druckdose (145) offenzuhalten, wenn die Druckdose (145) von der Aufnahme (170) aufgenommen ist;
eine Leitung (150), die mit der Aufnahme (170) verbunden ist, um das Pflegemittel zu dem Reinigungsraum (110) zu leiten;
ein Ventil (220), das in der Leitung (150) angeordnet ist, wobei ein Öffnungszustand des Ventils (220) durch ein Steuersignal (320) steuerbar ist;
einen Drucksensor (230), der zwischen der Aufnahme (170) und dem Ventil (220) mit der Leitung (150) gekoppelt ist, und welcher dazu ausgeformt ist, um ein Drucksignal (310) auszugeben, das einen in der Leitung (150) vorhandenen Druck repräsentiert; und
eine Steuereinrichtung (300), die ausgebildet ist, um unter Verwendung des Drucksignals (310) das Steuersignal (320) bereitzustellen.

2. Reinigungsgerät (100) gemäß Anspruch 1, wobei die Steuereinrichtung (300) ausgebildet ist, um das Steuersignal (320) zum Öffnen des Ventils (220) für eine von einer Größe des Drucksignals (310) abhängigen Zeitdauer bereitzustellen.

3. Reinigungsgerät (100) gemäß Anspruch 2, wobei die Steuereinrichtung (300) ausgebildet ist, um das Steuersignal (320) zum Öffnen des Ventils (220) für eine erste Zeitdauer bereitzustellen, wenn das Drucksignal (310) einen ersten Druck repräsentiert und um das Steuersignal (320) zum Öffnen des Ventils (220) für eine zweite Zeitdauer bereitzustellen, wenn das Drucksignal (310) einen zweiten Druck repräsentiert, wobei der erste Druck größer als der zweite Druck ist und die erste Zeitdauer kleiner als die zweite Zeitdauer ist.

4. Reinigungsgerät (100) gemäß einem der vorangegangenen Ansprüche, mit einem Rückschlagventil (225), wobei das Rückschlagventil (225) in der Leitung (150) zwischen der Aufnahme (170) und dem Drucksensor (230) angeordnet ist.

5. Reinigungsgerät (100) gemäß einem der vorangegangenen Ansprüche, wobei die Leitung (150) innerhalb des Reinigungsraum (110) mit einer Mehrzahl an Anschlüssen (235) für die medizinischen Instrumente verbunden ist.

6. Reinigungsgerät (100) gemäß einem der vorangegangenen Ansprüche, mit einem Druckluftanschluss (240), wobei die Leitung (150) über ein Druckluftventil (275) mit dem Druckluftanschluss (240) verbunden ist.

7. Reinigungsgerät (100) gemäß einem der vorangegangenen Ansprüche, mit einem Umwälzsystem zum Umwälzen von Reinigungsmittel unter Verwendung einer Pumpe (250), die mittels einer weiteren Leitung (255) mit der Mehrzahl an Anschlüsse (235) für die medizinischen Instrumente verbunden ist

8. Reinigungsgerät (100) gemäß einem der vorangegangenen Ansprüche, mit einem Verbindungselement (260) zum Verbinden der Pumpe (250) mit einer Korbankupplung (120) für einen Sprüharm (120).

9. Verfahren (600) zum Betreiben eines Reinigungsgeräts (100) gemäß den vorangegangenen Ansprüchen, wobei das Verfahren (600) folgende Schritte aufweist:
Ausgeben (601) des Drucksignals (310), das einen in der Leitung (150) vorhandenen Druck repräsentiert, unter Verwendung des Drucksensors (230), der zwischen der Aufnahme (170) und dem Ventil (220) mit der Leitung (150) gekoppelt ist;
Bereitstellen (603) eines Steuersignals (320) ansprechend auf das Drucksignal (310), unter Verwendung der Steuereinrichtung (300); und
Ansteuern (605) eines Öffnungszustand des Ventils (220), ansprechend auf das Steuersignal (320).
